# EUROPEAN PATENT APPLICATION

(11) **EP 4 481 750 A1**
(43) Date of publication of application: **25.12.2024**
(21) Application number: 23181243.9
(22) Date of filing: 23.06.2023
(51) Int. Cl.: G16H 10/20, G16H 10/60, G16H 70/40

(54) **PHARMACOVIGILANCE MONITORING AND MEDICAL REVIEW METHOD**

(71) Applicant: BOTh Analytics GmbH, 80687 München (DE)
(72) Inventor: Abdul-Ahad, Ayad, London, W5 2JF (GB); Snijder, Robert, 2317NL Leiden (NL); Mason, Philip, Stroud, GL5 4LF (GB)
(74) Representative: Bobbert & Partner Patentanwälte PartmbB

(57) **Abstract**

The invention comprises a method for pharmacovigilance monitoring and medical review according to the invention. The method comprises providing automated generation and/or management of study documents, providing risk analysis of patient safety, providing interactive graphs for main safety domains, providing in-depth study overviews, providing clickable statistical graphs, providing Burden of Therapy (BOTh) graphs, providing proactive warning signals and/or providing safety information management. The invention further comprises a digital storage medium, a computer program product and a computer program for executing the method.

## Description

The present invention relates to a method for pharmacovigilance monitoring and medical review. Furthermore, the invention comprises a digital storage medium, a computer program product and a computer program.

Carrying out a clinical trial requires high efficiency and effectiveness of data analysis in order to recognise and minimise treatment-related adverse events. Pharmacovigilance is focused on the detection, assessment, understanding and prevention and reporting of adverse effects of regulated products. The maintenance of safety data is a mandatory regulatory requirement. Hence, current pharmacovigilance systems focus on safety reporting, not medical monitoring and management.

It is an object of the present invention to suggest improved methods for pharmacovigilance monitoring and medical review. Furthermore, it is an object to propose an improved digital storage medium, computer program product and computer program for a computing device or computing unit.

The object according to the present invention is achieved by the method for pharmacovigilance monitoring and medical review having the features of claim 1. It is further achieved by the digital storage medium having the features of claim 13, the computer program product having the features of claim 14 as well as the computer program having the features of claim 15.

In all of the aforementioned and following statements, the use of the expression "may be" or "may have", etc., is to be understood synonymously with "preferably is" or "preferably has", etc., respectively, and is intended to illustrate an embodiment according to the present invention.

Whenever numerical words are mentioned herein, the person skilled in the art shall understand them as indications of numerical lower limits. Hence, unless this leads to a contradiction evident to the person skilled in the art, the person skilled in the art shall comprehend for example "one" as encompassing "at least one". This understanding is also equally encompassed by the present invention as the interpretation that a numerical word, for example, "one" may alternatively mean "exactly one", wherever this is technically possible in the view of the person skilled in the art. Both meanings are encompassed by the present invention and apply herein to all used numerical words.

Whenever the terms "programmed" or "configured" are mentioned herein, these terms are interchangeable.

The method for pharmacovigilance monitoring and medical review according to the invention comprises the step(s) of:
- providing automated generation and/or management of study documents;
- providing risk analysis of patient safety;
- providing interactive graphs for main safety domains;
- providing in-depth study overviews;
- providing clickable statistical graphs;
- providing Burden of Therapy (BOTh) graphs;
- providing proactive warning signals; and/or
- providing safety information management.

The method according to the invention preferably aims at improving patient safety and health as well as avoiding adverse events.

In one alternative, the method of claim 1 comprises automated generation and/or management of clinical study documents, in particular essential pharmacovigilance-related documents, such as patient Serious Adverse Event (SAE) reports. The method may automate the reporting of studied patient populations and individual patients, in particular patients with SAEs. Advantageously, this integration of document generation and management may streamline processes for users, enabling them to efficiently monitor and address any safety concerns that may arise during the course of the clinical study.

In another alternative, the method of claim 1 provides risk analysis of patient safety under treatment, in particular ensuring vigilant monitoring and care. This feature preferably allows for early detection and mitigation of potential safety issues.

Furthermore, in an alternative, the method of claim 1 provides interactive graphs for main safety domains, such as adverse events, laboratory data and/or vital signs. These graphs may advantageously provide clear visualisation of complex data, enabling stakeholders to quickly understand and interpret trial results.

In an additional alternative, the method of claim 1 provides in-depth study overviews. The in-depth study overviews preferably include demographic data, disposition information and/or visit overviews. These may advantageously enable an improved understanding of the trial landscape.

In an alternative, the method of claim 1 provides clickable statistical graphs. The method according to the invention generates clickable statistical graphs that preferably allow end users to drill down from high-level information to specific areas of interest or patient-level data.

In an alternative, the method of claim 1 provides Burden of Therapy (BOTh) graphs. The method according to the invention generates unique BOTh graphs for adverse events. Advantageously, these BOTh graphs may provide insights into the development of adverse events following study start-up by combining frequencies, start dates and/or duration in a single graphical overview.

The BOTh graphs may involve the use of a clinical study database containing individual data entries for treatment-emergent adverse events (TEAEs) per patient per day. This approach may allow for the generation of a graph displaying the number of patients with TEAEs on each study day. TEAEs may be plotted against the study day and the worst severity may be considered in cases where multiple events with varying severity are reported on the same day. End dates for ongoing TEAEs or those with missing end dates may be replaced by the patient's last study day.

In an alternative, the method of claim 1 provides proactive warning signals. The proactive warning signals may be generated for any safety concerns that may arise during the clinical trial, which may advantageously enable prompt intervention and risk mitigation.

In an alternative, the method of claim 1 provides safety information management. Preferably, the system streamlines regulatory compliance and/or enhances the efficiency of safety information management, in particular through automated CIOMS (Council for International Organizations of Medical Sciences) reporting.

According to the invention, a digital, in particular nonvolatile, storage medium, in particular in the form of a machine readable carrier, in particular in the form of a floppy disk, CD, DVD or an EPROM, in particular with electronically or optically readable control signals, is configured to interact with a computing device and/or computing unit, such that the machine implemented steps of the method according to the invention are prompted.

A computer program product according to the invention comprises a program code saved on a machine-readable carrier for prompting the machine implemented steps of the method according to the invention when the computer program product runs on the computing device and/or the computing unit.

A computer program according to the invention comprises a program code for prompting the machine implemented steps of the method according to the invention when the computer program runs on the computing device and/or the computing unit.

In some embodiments, the method utilises a cloud-based infrastructure. Preferably, the method is implemented at least partly on a secure, cloud-based infrastructure. This may advantageously guarantee data privacy and/or compliance with relevant data protection regulations.

In some embodiments, the invention provides a data analysis tool designed to improve the way clinical trial data is processed, visualised and interpreted.

In some embodiments, the invention comprises a data analysis platform that aims to improve the efficiency and effectiveness of clinical trial data analysis by leveraging its distinctive capabilities and features. The platform offers a range of functionalities, including, but not limited to, advanced data visualisation, customisable reporting and integration of novel methodologies, such as a Burden of Therapy (BOTh) approach for assessing treatment-related adverse events.

In some embodiments, the BOTh approach helps to identify adverse events experienced by patients during clinical trials.

In some embodiments, the method comprises providing automated reporting of SAE. In particular, the method may generate and manage essential pharmacovigilance-related study documents, such as patient Serious Adverse Event (SAE) reports and CIOMS (Council for International Organizations of Medical Sciences) reports.

In some embodiments, the method comprises an analysis of the safety burden faced by patients. In particular, the BOTh approach enables users to visualise and analyse the safety burden faced by patients, which advantageously may provide a more comprehensive understanding of the impact of treatment-related adverse events.

In some embodiments, the method comprises reading from a database containing data entries related to treatment-emergent adverse events (TAEAs). The BOTh graphs may involve the use of a clinical study database containing individual data entries for treatment-emergent adverse events (TEAEs), preferably per patient per day. This approach may allow for the generation of a graph displaying the number of patients with TEAEs on each study day. TEAEs may be plotted against the study day and the worst severity may be considered in cases where multiple events with varying severity are reported on the same day. End dates for ongoing TEAEs or those with missing end dates may be replaced by the patient's last study day.

In some embodiments, the method comprises providing a graph containing weighted TEAEs. Preferably, to better analyse the safety burden experienced by patients, consistent weighting is applied to TEAE severity, in particular across each study arm. This may result in a graph that displays the weighted TEAEs per day, with mild, moderate, and severe TEAEs weighted, for example, as 1x, 2x, and 3x TEAEs per event per day, respectively.

In some embodiments, the method comprises computing an area under curve (AUC) per patient. The BOTh approach preferably allows for the calculation of an Area Under Curve (AUC) per patient, which represents the burden estimate for each patient.

By preferably employing an ANCOVA model with the burden estimate as the dependent variable and treatment as the independent variable, users can estimate the treatment effect of the BOTh approach.

In some embodiments, the method comprises user authentication. User authentication may result in better data security and privacy. Also, settings and preferences may be associated with each user account, such that different users will have their own settings and preferences upon login, which may increase productivity.

In some embodiments, the method comprises filtering for variables. Patient data can be analysed using filters. Filters chosen may be applied to any graphic or tabular data displayed.

In some embodiments, a central hub may provide users access to crucial information and insights about a selected clinical study or studies. Designed to provide a comprehensive, high-level overview of the current status of a given study, the central hub may display a range of key data points, including but not limited to, an overview of adverse events, visit-by-visit counts of patients in real-time and demographic characterisations of a study population.

In some embodiments, the central hub may also be expanded to accommodate multiple studies simultaneously. This multi-study functionality may allow users to monitor and/or manage several studies concurrently, in particular providing them with a comprehensive and/or real-time snapshot of some or all of their clinical trial portfolio. Advantageously, this approach to study management may enable users to make informed decisions across various studies and ensures that potential issues may be identified and addressed promptly and effectively.

In some embodiments, the central hub may serve as a powerful and versatile tool for users and may offer them essential insights and/or functionalities to effectively manage and/or analyse their clinical trial data. The central hub preferably provides a user-friendly interface that seamlessly integrates data visualization, real-time monitoring and/or document generation capabilities.

In some embodiments, the method comprises providing a box chart. In some embodiments, a graph selector generates a highly adaptable graph, in particular with user-defined arms, categorised stacks and/or interactive drill-down capabilities. This functionality preferably enables users to analyse data in a more granular fashion, facilitating informed decision-making.

The method may provide a Hy's Law graph, preferably with labelled segments, clickable points and/or tables displaying matching subjects. Advantageously, this may provide users with a visual representation of critical data points.

Furthermore, in some embodiments, box charts may allow users to select lab tests, layout configurations and/or variable-based graph sets. This may enhance the method's overall adaptability.

In some embodiments, the method provides a graph selector, which may allow users to view box charts of measurements, in particular of vital sign measurements. In some embodiments, graphs are generated with normal ranges denoted discernibly. In some embodiments, the method may generate graph sets, lab tests and/or layout configurations to afford users a tailored analysis experience. Furthermore, a list feature includes visually enhanced bars that indicate normal, low and/or high values. This advantageously may enhance the methods applicability and use.

In some embodiments, the method may provide a graph selector, which presents a Sankey chart. This may demonstrate the flow of subjects through various study stages. Additionally or alternatively, a vertical bar chart may demonstrate visit numbers against dates, preferably segmented by disposition. These features may advantageously provide users with an easy overview of subject progression, facilitating efficient data analysis. Additionally, a table with subject data may be generated for more detailed examination.

In some embodiments, the method may generate a collection of graphs displaying key demographic information, preferably with interactive elements, in particular enabling users to view matching subjects in a table. These graphs may include horizontal age-based bar charts and/or pie charts for sex, study arm, race, ethnicity and/or site. The method's interactive capabilities and/or visualisation options may advantageously make it an asset for users seeking to analyse demographic data.

In some embodiments, the method may provide a graph selector, which provides users with a vertical bar chart, preferably displaying visit numbers by date, in particular segmented by visit type. Users may be able to interact with the chart to view matching subjects in a table, advantageously allowing swift and accurate data analysis.

In some embodiments, the method provides patient profiles with a comprehensive and detailed overview of individual patients' safety data within a clinical trial, preferably over time. Preferably, the patient profiles are accessible from any graph and/or location within the platform. Advantageously, these patient profiles may offer users an intuitive and dynamic view of safety events over time, enabling them to analyse the data in a contextually meaningful manner.

Preferably, the patient profiles comprise a fixed time axis, which harmonises the display of safety data across various domains, such as laboratory results, vital signs and/or adverse events (AEs). This time-based alignment of data may allow users to easily discern patterns and relationships between different events, potentially helping them identify safety concerns and assess their impact on the patient's well-being.

In some embodiments, patient profiles offer users a view of individual patients' safety data within a clinical trial. The method's dynamic generation of information, combining temporal alignment and intuitive visualisations, may allow users to effectively analyse and interpret safety events, contributing to improved decision-making and enhanced patient care.

In some embodiments, the method comprises providing an artificial intelligence interface. In some embodiments, a graph selector offers a wide variety of report types, in particular SAE reports, AI-driven question-and-answer interfaces, general reports and/or R and Python program outputs. The method may also allow users to generate downloadable PDF and/or Microsoft Word format reports, which may advantageously cater to a diverse range of user requirements and preferences. The AI interface may be provided as a conversational AI, allowing the user to interact with the method in natural language. The AI interface may be implemented as a neural network, in particular as a large language model.

In some embodiments, the method provides access to study notes and/or key files, which may streamline the organisation and management of crucial data. A transparent note area and/or modal window may facilitate note viewing and downloading, which may enhance usability.

In some embodiments, the method comprises a menu, study selection icons, lab alerts, global filter builders and/or an icon ribbon for SDTM domains.

The present invention is explained in the following examples and using accompanying figures. The following applies in the figures:
Fig. 1 shows the user interface of one implementation of the method according to the invention in a first representation showing adverse events in a BOTh graph;
Fig. 2 shows the user interface of one implementation of the method according to the invention in a second representation showing vital signs, in particular BMI, in a box plot;
   and
Fig. 3 shows the user interface of one implementation of the method according to the invention in a third representation showing adverse events in a sunburst.

In one example of the method according to the invention, a data analysis tool incorporates the Burden of Therapy (BOTh) approach, which herein emphasises the adverse events experienced by patients during clinical trials. In this example, the BOTh approach enables users to visualise and analyse the safety burden faced by patients, providing a more comprehensive understanding of treatment-related adverse events.

In this example, the BOTh approach involves the use of a clinical study database containing individual data entries for treatment-emergent adverse events (TEAEs) per patient per day. This approach allows for the generation of a graph displaying the number of patients with TEAEs on each study day. TEAEs are plotted against the study day, and the worst severity is considered in cases where multiple events with varying severity are reported on the same day. End dates for ongoing TEAEs or those with missing end dates are replaced by the patient's last study day.

To enhance the clarity of the visual representation in this example of the method according to the invention, colours are used to correspond with TEAE severity, enabling easy discrimination of TEAEs of differing severity levels in the graph. Additionally or alternatively, to accurately depict the safety burden experienced by patients, consistent weighting is applied to TEAE severity across each arm. This may result in a graph that displays the weighted TEAEs per day, with mild, moderate, and severe TEAEs weighted as 1x, 2x, and 3x TEAEs per event per day, respectively.

In one example, the BOTh approach also allows for the calculation of an Area Under Curve (AUC) per patient, which represents the burden estimate for each patient. By employing an ANCOVA model with the burden estimate as the dependent variable and treatment as the independent variable, users can estimate the treatment effect on the BOTh. By including baseline characteristics as covariates, users can adjust the treatment difference estimates for potential confounding factors, providing a more accurate assessment of the treatment's impact on the BOTh.

The integration of the Burden of Therapy approach makes the method a unique and valuable tool for analysing and visualising adverse events in clinical trials. This innovative approach offers users a more comprehensive understanding of the safety burden experienced by patients.

In an example, tooltips are shown whenever hovering over icons to describe what they indicate or what their functionality is.

In an example, the system requires the user to log in, so that it can only be used by those with a username and password. There may be different categories of user with divergent permissions. Access to data may be controlled via definitions in a JSON file as to what data certain categories of users are permitted to access.

In one example, the user interface is written in JavaScript as a Web Application. The user interface may make calls to a server via an Application Programming Interface (API).

In one implementation, the server is written in JavaScript node.JS. An API is implemented with a number of possible calls.

Some or all of the following API calls may be implemented in an embodiment of the invention:
- Getcsvdata - takes a file name as a parameter and then imports it as a CSV file, then returns the data as a JavaScript object.
- Getfile - takes a file name as a parameter and then imports it as a CSV file, then returns the contents as text.
- Sql - takes the name of the database as the first parameter and then the SQL code to run as the 2nd parameter. The result is returned as a JavaScript object with a message part showing any messages from running the SQL and a data part with rows of data. This runs as a HTTP GET request.
- Sqlpost - same as Sql, but runs as a HTTP POST request. The advantage is that there are fewer limitations such as size of code passed in.
- Note - takes some SQL code which is then applied to the URL notes database, where notes are stored for each screen (represented by a URL) and database (or study).
- Getdata - takes a file name as a parameter and then returns the text of that file.
- Dir - takes a folder as a parameter, and then returns the content of the folder showing filenames, size, dates.

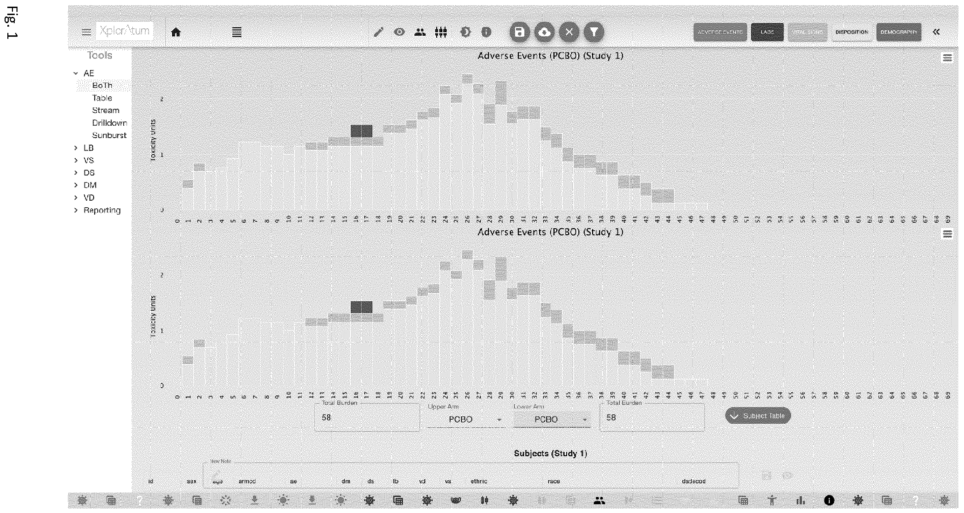

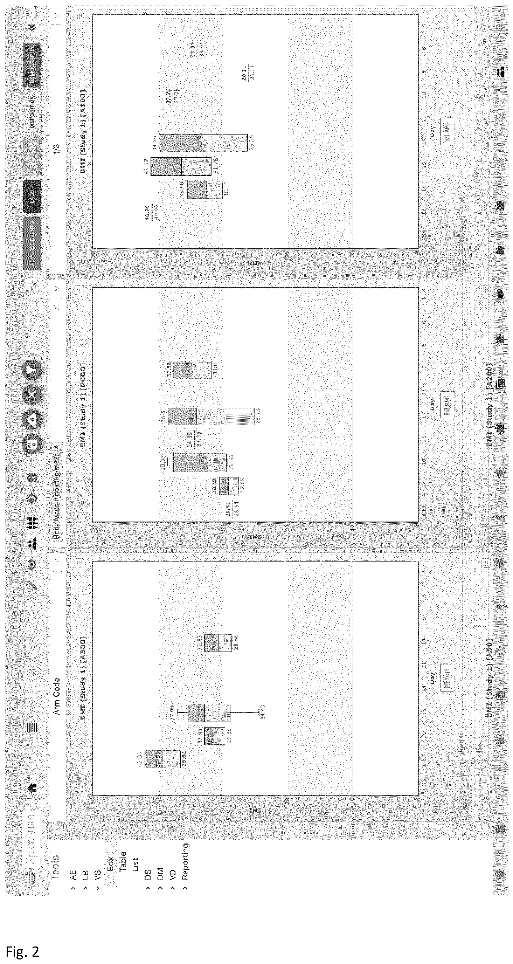

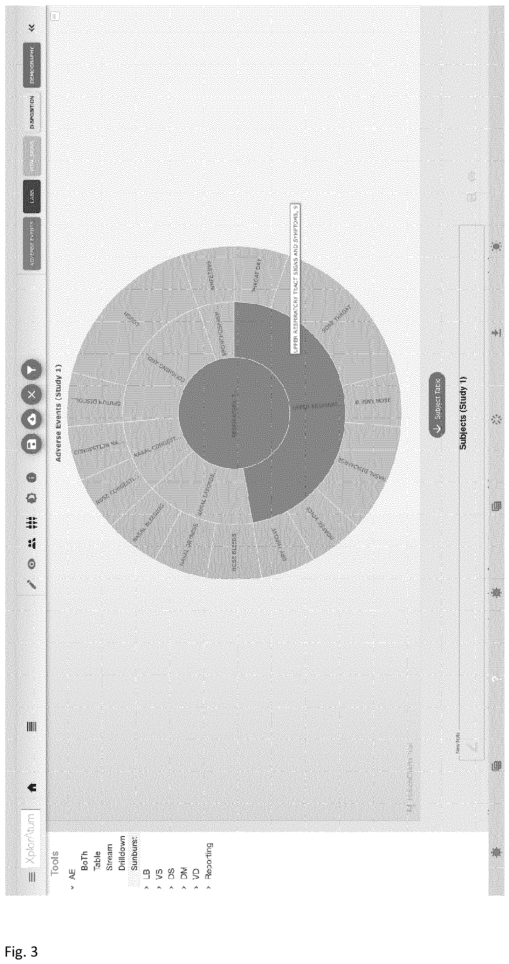

In one implementation, the home screen shows some or all of the following items:
- Name of study ("Study 1" as shown in Figs. 1 to 3) and the database used.
- First and last dates of visits made by subjects.
- Calendar chart of number of subject visits per day in a grid showing month vs day of the week. A heatmap may be used to show the number ranges, where darker colours indicate higher number ranges, e.g., 0-5, 6-10, 11-15, etc.
- A series of ovals is shown with information about AEs (Adverse Events). Each oval shows some information about AEs. If a user clicks on one of the ovals, it will create a global filter applicable for what was clicked on. For instance, clicking on "number of serious AEs" also creates a filter showing only serious AEs. The series of ovals may include some or all of the following:
   - Number of subjects that have an AE and the percentage, e.g., x of y (z%) subjects have an AE
   - Total number of AEs in study
   - Number of non-serious AEs in study
   - Number of un-related AEs in study
   - Number of possible related AEs in study
   - Number of probably related AEs in study
   - Number of serious AEs in study
- A series of donut charts, which comprise some or all of the following:
   - Number of AEs by seriousness
   - Number of AEs by severity
   - Number of AEs by relatedness
- Global filter system, which may comprise some or all of the following:
   - Variables to filter on are defined in a JSON file, to make it easily configurable
   - Filters chosen are applied to any graphic or tabular data displayed
   - Filters used are shown in titles or footnotes of graph or table displayed
- Tools are arranged based on Study Data Tabulation Model (SDTM) domains, including some or all of the following (s. Figs. 1 to 3):
   - e.g., AE, LB, VS, DS, DM, VD
   - Quick access buttons for selected SDTM domains to open the most common graph/table for that area.
- Toolbars/menus appear at top, left and right - plus there is a note area at the bottom of the screen (s. Fig. 1 to 3)
   - Top toolbar has the following icons:
      - main menu (three parallel lines) is available by clicking on left most icon. It is a drop-down menu that can take you to many places in the tool.
      - a logo (in Figs. 1 to 3: "Xploratum") has a tooltip to show what you are currently logged in as
      - a home icon (symbol of a house) takes you to the home screen, which shows an overview of the currently selected study
      - another icon (5 parallel lines) will allow displaying of key documents from a study such as the Protocol & SAP
      - Several icons to:
         - make a note (symbol of a pencil) for this current view, which is connected to the study and the screen (being report/table/graph) being viewed
         - view all notes (symbol of an eye) related to the current study and screen
      - An icon (symbol of two people) to show the subject overview shows a table of subjects. On each row we get some basic demographic data such as sex, age, ethnicity & race, but also ticks under SDTM domains to show in which domains we have data for the subject. Under the AE column, we also use some icons to show if there are any serious AE, probably related, and other interesting things. Counts of the number of AEs of various types are shown in columns (serious, moderate, severe, possibly related, probably related, died, ongoing). Also, disposition is shown, such as whether a subject withdrew from a study. Arm of study is also shown. Clicking on a row takes you to the patient profile for that subject in another tab.
      - a preferences icon opens the preferences screen that shows and allows editing of:
         - lab limits in categories with low and high values
         - screen size
         - vital signs limits
         - drill down path to use for adverse events graphs that support drill down
      - an icon (symbol of sun and moon) toggles between light and dark mode
      - an info icon ("i") will display information about the current screen
      - a series of four coloured icons relate to global filters
         - The first icon (symbol of a floppy disk) will save the currently selected filters to one of several slots. If a filter is in effect, then the colour of this icon will change to an alternate colour to indicate it can be clicked on and a filter saved.
         - the next icon (cloud with arrow pointing down) loads selected filter from one of the saved slots
         - the next icon (cross) removes any filter currently in effect. If a filter is currently in effect, then the colour of this icon is displayed with an alternate colour to indicate that it could be clicked to remove the filter in effect.
         - the final icon (symbol of a filter) opens a modal window to allow selection of the global filter
      - a series of colourful buttons (adverse events/labs/vital signs/disposition/demography) are shown, which link to the most common screen within each of several SDTM areas. For example, click on the adverse events button will open the AE menu on the left and show the BOTh screen. The buttons displayed and which screens they open are configurable via a JSON file.
   - Left menu shows SDTM areas which can be expanded (AE (expanded in Figs. 1 and 3), LB, VS (expanded in Fig. 2), DS, DM, VD, Reporting) to show screens that can be selected to display content in the central area. There are also some other categories that can be shown such as Reporting, where additional screens are grouped which do not directly relate to an SDTM area.
      - each SDTM area has a range of screens which can be selected by clicking.
      - each SDTM area has a table screen which will simply show the SDTM table in a flexible table object.
      - when a subject id is shown, the table will allow clicking on that to open that subject's Patient Profile on another tab.
      - an icon is shown to allow the table to be downloaded as a CSV.
      - a switch is shown labelled Subjects, which affects how the filters are used. By default, the switch is on, meaning that any filter in effect will result in a set of subjects and all rows in the table will be shown for that set of subjects. If the switch is off, then only rows in the table that match the corresponding filters will be shown. For example, if serious AEs were selected in the filter, then if the switch is on then the AE table would show all rows that have a patient with any serious AE. However, if the switch was off then only rows with a serious AE would be shown.
   - AE - Adverse events
      - shows a BOTh AE graph (s. Fig. 1). Indicates the total burden for the top and bottom graphs. Allows choosing which arm to use for each of the top and bottom graphs. Clicking on items in the legend will toggle them on/off in both the top and bottom graphs.
      - stream graph shows the number of AEs in effect day by day. Clicking on the graph will drill down on what was clicked on using the AE drill down hierarchy. What was drilled down on is always shown in a subtitle. Once drilled down you see a legend showing the level you are at in the hierarchy and providing icons to drill up a level or return to the top.
      - Drilldown graph shows number of AEs and number of subjects for each category in the drill down hierarchy. Clicking on a bar will drill down a level. Legend can be clicked to toggle bars on and off. Once drilled down you see a legend showing the level you are at in the hierarchy and providing icons to drill up a level or return to the top.
      - Sunburst graph provides an overview of AEs initially showing all categories in a drill down hierarchy at once (s. Fig. 3). Clicking on something will drill down and show a new sunburst for what was clicked. Clicking the centre of sunburst goes up a level if possible. A table of subjects is shown under the graph which represents all those subjects currently shown by the sunburst.
   - LB - Labs
      - shows a BOTh Labs graph which has different arms shown in the top and bottom, which can be changed by user. Bars are broken into stacks by the lab category. Clicking on a bar will drill down on that lab category and then show a stack of lab tests in each bar. Clicking on a lab test drills down to show the graph just for that lab test. The table below graph always shows what was clicked on and allows linking to patient profile if a row is clicked on.
      - Hy's Law graph with 3 interesting segments labelled. Points can be clicked on to show a table with the matching subject below. Table can be clicked on to link to patient profile.
      - Box charts can be shown which take the values or one or more lab tests and show a box plot of the values versus the day of treatment. If limits are available for the first lab test chosen, then its normal range will be shown on each graph with a green background shading.
         - A selector allows a set of graphs to be produced making one for each value of the variable chosen such as arm code, sex, age, subject id, race, ethnicity or site (defined in a JSON file so the variables can be changed).
         - A selector allows choosing one or more lab tests which will be shown on box plots.
         - A selector allows choosing how to layout graphs on the screen - so you can have 1, 2, 3, 4, 6 or 12 graphs across.
   - VS - Vital Signs
      - Box charts can be shown which take the values or one or more vital signs measurements and show a box plot of the values versus the day of treatment (s. Fig. 2). If limits are available for the first lab test chosen, then its normal range will be shown on each graph with a green background shading.
      - A selector allows a set of graphs to be produced making one for each value of the variable chosen such as arm code, sex, age, subject id, race, ethnicity or site (defined in a JSON file so the variables can be changed).
      - A selector allows choosing one or more lab tests which will be shown on box plots.
      - A selector allows choosing how to layout graphs on the screen - so you can have 1, 2, 3, 4, 6 or 12 graphs across.
      - Vital signs list shows all the measurements taken for subjects but with coloured bars behind each figure to indicate if it is normal (yellow), lower than the lower limit of normal (blue) or higher than the upper limit of normal (red). The length of the bar shows the relative magnitude of the value, so the shortest bar in the column is the lowest value and longest bar is the highest. If no limits are available then the length of the bar is still used to indicate magnitude, but the colour used is, e.g., white.
   - DS - Disposition
      - A Sankey chart shows the number of subjects in various states throughout the study. It shows the flow, e.g., from screening to screened to randomised to safety to exposed. Then it will show what arm of study subjects move into and then what their final disposition is (e.g., completed, withdrew).
      - A vertical bar chart shows number of visits against date and segments each bar by the disposition. The legend can be used to select what is shown of graph. A table is shown under the graph with subject's data for the last segment of graph that was clicked on.
   - DM - Demography
      - A collection of graphs is shown for the key information. Clicking on a part of any graph will show the subjects matching what was clicked on in a table below. Rows in that table can be clicked on to open the subject's patient profile.
         - Horizontal bar chart of age of subjects broken into 5-year groups.
         - Pie chart showing the number of subjects of each sex.
         - Pie chart showing the number of subjects in each arm.
         - Pie chart showing the number of subjects in each race.
         - Pie chart showing the number of subjects in each ethnicity.
         - Pie chart showing the number of subjects in each site.
   - VD - Visit Data
      - Vertical bar chart showing the number of visits by date, with each bar segmented by the type of visit. Clicking on part of a bar will show a table below of matching subjects.
   - Reporting
      - Serious AE reports have a collection of reports that can be produced from the serious AE data. Currently, there are three types, although many more can be added. Selecting a report will automatically generate a Microsoft Word file containing the report, which then allows it to be modified by the medical writer.
      - An Artificial Intelligence interface allows asking questions and getting answers. This may operate on the data in the study.
      - General reports are available
         - R programs can be run and the output shown.
         - Python programs can be run and the output shown.
         - PDF reports can be produced.
         - Microsoft Word format reports can be produced.
      - Notes screen displays all the notes available for the current study that is selected. Notes can also be seen by going to a particular screen and looking at notes for that screen, but this provides a central point for accessing all notes.
      - Diagrams can be generated from certain data in the study.
      - Files screen displays some key files that are available for the study.
- Right menu appears when tool is selected from toolbar
   - It is an accordion menu where a selection will open up the content for that tab.
   - Studies are selected using icons at the top, allowing the system to be used with multiple studies.
   - Lab alerts are shown by choosing that accordion item
      - A table is displayed showing a list of subjects with the lab test, value, date and time.
      - Clicking on a subject number opens the patient profile in another tab.
      - A link is shown for each subject that opens that patient's lab results for the category of that lab test, and then allows switching to look at the lab test values for other subjects for that test.
   - All tables can be viewed from one of the tabs, so that all data in the database is available via this tool. Clicking on one of the tables will open a view of the data.
   - Subject overview shows a slightly narrower version of the subject overview (previously mentioned)
   - Global filter builder is available here to see and change what filters are in effect.

   - Note area at the bottom of screen.
      - It is usually transparent, so it can be seen lightly but you can see content on the screen behind it
      - Moving the mouse over it makes it non-transparent
      - You can click in the New Note box (s. Figs. 1 to 3) and then enter a note. Clicking on the save icon will save what you have entered into the notes database for the current screen and study.
      - Clicking on the view notes icon opens up a modal window to show all the notes recorded for this study and screen.
         - You can download notes from here to a CSV for use in EXCEL and other products
   - Icon ribbon at the very bottom of screen.
      - Every time the user selects a different screen, its icon is added to the ribbon at the bottom. This allows you to keep track of where you have been and, by clicking on an icon, to go back to a previous screen. Colours are used to classify the icons into different SDTM domains, which also match the buttons at the top right for colours used for SDTM domains.
   - Patient Profiles
      - A patient profile can be shown for any patient. Most of the time, where there is a subject id shown in the system, you are able to click on it and go to the corresponding patient profile.
      - At the top of the patient profile common demography info is shown, such as: age, sex, race and ethnicity. We also show:
         - the arm of study (if available)
         - whether exposed to study drug
         - whether randomised
         - whether in safety population, PK and PD set
         - if study was completed
   - A grid is shown that shows AE info, vital signs and lab info. Days of treatment are shown across the top of grid. The table can be scrolled horizontally to look through data for each day of treatment.
      - AE info is shown with the days that the AE covered and its severity. The severity is colour coded as well as shown in text. It provides a simple view of when AEs occurred. Hovering over the severity text shows more key information about that AE.
      - Clicking on the Adverse Event link opens up a modal window with general info on the subject at the top, followed by all the available info for each AE after that.
      - Vital signs are shown next, with any vital sign measurements shown under the day of treatment.
      - Labs data is shown next, the categories of lab data may be shown with icons that indicate how they compare to normal values.
         - A green tick indicates that all the lab values are within the normal range(s)
         - A yellow circle with a cross indicates that some lab values exceed the upper level of normal
         - A red circle with a cross indicates that some of the lab values exceed twice the upper level or normal
         - Clicking on a lab category opens up a modal window which has demography info at the top, and then shows a grid with:
            - Each lab test is listed
            - Unit of result
            - Lower limit of normal
            - Upper limit of normal
         - Each day of treatment that had lab tests done
         - Values of lab tests are shown in different colours if outside the normal range
         - Orange is over upper level of normal
         - Red is over twice upper level of normal
         - Blue is under lower level of normal
      - Next a BOTh graph is shown for the subject, showing the burden of therapy. A link appears higher up the screen which can be clicked on to jump down to the BOTh graph. This is to make it clear there is a useful graph below, since you usually need to scroll to see it.

In an exemplary embodiment of the method according to the invention, the method is a web-based application written in JavaScript (React) that makes use of CDISC SDTM compliant data, to provide interactive graphs, tables and reports. It allows to follow the natural workflow process for medical monitoring and pharmacovigilance review. A flexible filter system allows targeting the population of interest. A high-level overview of data and zoom down into the details of subgroups and patient-level information is implemented to assist in medical monitoring and subject management. A powerful patient profiler screen is available to examine all aspects of safety of a specific subject, based on the available data.

Detailed listings are provided in an exemplary embodiment. Data can be exported to other systems at any point. Lab alerts can be generated and can be checked off as they are dealt with. The time variations of safety indicators from the first to the last day can be monitored as all relevant graphs, including the patient profiles, may display the dynamics over time. Specifically, the methodology Burden of Therapy (BOTh), a unique way of monitoring AEs over time can be an integral part of the application. As required by pharmacovigilance, SAE reporting can be as much as possible automated. Each step can be saved and notes can be written.

In an exemplary embodiment of the method according to the invention, the landing page offers users the ability to generate and manage essential pharmacovigilance-related study documents, such as, e.g., patient Serious Adverse Event (SAE) reports and/or CIOMS (Council for International Organizations of Medical Sciences) reports.

In an example, in a labs graph selector, a highly adaptable graph with user-defined arms, categorised stacks and/or interactive drill-down capabilities is generated. This functionality may enable users to analyse data in a more granular fashion, facilitating informed decision-making. The tool may also generate a Hy's Law graph, preferably with labelled segments, clickable points and/or tables displaying matching subjects, such that users can be provided with a visual representation of critical data points. Moreover, the generation of box charts may allow users to select lab tests, layout configurations and/or variable-based graph sets, preferably enhancing the tool's overall adaptability.

In an exemplary embodiment, a vital signs graph selector in may allow users to view box charts of vital sign measurements, preferably with normal ranges denoted by coloured background shading. Customisable graph sets, lab tests and/or layout configurations may allow users a tailored analysis experience. Furthermore, a vital signs list feature includes bars, which are preferably color-coded, that indicate normal, low and/or high values, which may enhance the tool's utility and/or visual appeal.

In an exemplary embodiment, a disposition graph selector may present a Sankey chart, preferably illustrating the flow of subjects through various study stages. Additionally or alternatively, a vertical bar chart, preferably displaying visit numbers against dates, more preferably segmented by disposition, may be implemented. These features may provide users with an at-a-glance view of subject progression, preferably facilitating efficient data analysis. Additionally, a table with subject data may be available for more detailed examination.

In an example, laboratory and/or vital sign data, the patient profile's front page highlights values that may fall within or outside the normal range, preferably using color-coded indicators to signify deviations (e.g., above, much above, below, or much below normal range). This visual representation may enable users to quickly identify any abnormalities, while preferably also offering the option to access more detailed information through a graphical table, e.g., by clicking on the lab and/or vital signs header.

In an example, adverse events may be presented in a graphical format, preferably displaying the start and/or stop times and/or the severity of each event. This visualisation may facilitate a clear understanding of the temporal relationships between different AEs and/or their potential impact on the patient.

In an example, the patient profile also includes the individual patient's Burden of Therapy (BOTh) graph. This personalised representation of the patient's safety experience within the trial may offer users a unique perspective on the cumulative impact of treatment-related events on the patient's overall well-being.

In an example, patient profiles offer users an unparalleled view of individual patients' safety data within a clinical trial. The platform's dynamic presentation of information, combining temporal alignment and/or intuitive visualisations may allow users to effectively analyse and interpret safety events, preferably contributing to improved decision-making and/or enhanced patient care.

In an example, a reporting graph selector may offer a wide variety of report types, preferably including SAE reports, AI-driven question-and-answer interfaces, general reports and/or R and/or Python program outputs. The tool may also allow users to generate downloadable PDF and/or Microsoft Word format reports, preferably catering to a diverse range of user requirements and/or preferences.

In an example, a centralised screen provides access to study notes and key files, preferably streamlining the organisation and/or management of crucial data. A transparent note area and/or modal window may facilitate note viewing and downloading, preferably enhancing the tool's usability.

In an example, a user interface may feature an accordion menu, study selection icons, lab alerts, global filter builders and/or an icon ribbon for SDTM domains, preferably making it highly intuitive and user-friendly.

## Claims

1. A method for pharmacovigilance monitoring and medical review, wherein the method comprises at least one of:
- providing automated generation and/or management of study documents;
- providing risk analysis of patient safety;
- providing interactive graphs for main safety domains;
- providing in-depth study overviews;
- providing clickable statistical graphs;
- providing Burden of Therapy (BOTh) graphs;
- providing proactive warning signals; and
- providing safety information management.

2. The method according to claim 1, wherein the method utilises a cloud-based infrastructure.

3. The method according to one of the previous claims, wherein the method comprises an analysis of the safety burden faced by patients.

4. The method according to one of the previous claims, wherein the method comprises reading from a database containing data entries related to treatment-emergent adverse events (TAEAs) and/or providing a graph containing weighted TEAEs.

5. The method according to one of the previous claims, wherein the method comprises computing an area under curve (AUC) per patient.

6. The method according to one of the previous claims, wherein the method comprises employing an ANCOVA model with the burden estimate as the dependent variable and treatment as the independent variable.

7. The method according to one of the previous claims, wherein the method comprises user authentication.

8. The method according to one of the previous claims, wherein the method comprises filtering for variables.

9. The method according to one of the previous claims, wherein the method comprises providing a box chart.

10. The method according to one of the previous claims, wherein the method comprises providing a Hy's Law graph.

11. The method according to one of the previous claims, wherein the method comprises providing a Sankey chart.

12. The method according to one of the previous claims, wherein the method comprises providing an artificial intelligence interface.

13. A digital storage medium, in particular in the shape of a floppy disk, CD or DVD or EPROM, with electronically readable control signals, configured to interact with a computing device and/or a computing unit, such that the machine implemented steps of the method according to one of the claims 1 to 12 are performed.

14. A computer program product with a program code stored on a machine-readable carrier for executing the machine implemented steps of the method according to one of the claims 1 to 12, when the computer program product is running on the computing device and/or the computing unit.

15. A computer program with a program code for prompting the machine implemented steps of the method according to one of the claims 1 to 12, when the computer program is executed on the computing device and/or the computing unit.
